# EUROPEAN PATENT APPLICATION

(11) **EP 3 098 604 A1**
(43) Date of publication of application: **30.11.2016**
(21) Application number: 16170604.9
(22) Date of filing: 20.05.2016
(51) Int. Cl.: G01N 33/543, G01N 33/66

(54) **GEL FOR SENSOR AND SENSOR**

(30) Priority: 22.05.2015 JP 2015104274
(71) Applicant: Seiko Epson Corporation, Tokyo 160-8801 (JP)
(72) Inventor: Yoshioka, Satomi, Tokyo, 392-8502 (JP); Yagi, Hiroshi, Tokyo, 392-8502 (JP)
(74) Representative: Hoffmann Eitle

(57) **Abstract**

A sensor includes a stimulus-responsive gel which reversibly expands or contracts in response to a given stimulus, an electrically conductive gel containing electrically conductive particles constituted by a material containing an electrically conductive substance, and an electrode connected to the electrically conductive gel. The electrically conductive particles preferably have an average particle diameter of 10 nm or more and 1000 µm or less.

## Description

### BACKGROUND

### 1. Technical Field

The present invention relates to a gel for a sensor and a sensor.

### 2. Related Art

At present, as a method for obtaining in vivo biological information, a biochemical test in which the composition of the blood obtained by blood collection is examined is generally performed. This test is mostly performed in medical institutions. Above all, a blood glucose sensor has been widely used in diabetic patients, and also a simple lactic acid sensor is getting widely used in athletes.

However, both are test methods involving blood collection using an invasive technique.

On the other hand, as a method using a non-invasive technique, a sensor targeting a component of sweat has been studied (see, for example, Wearable Technology for Bio-Chemical Analysis of Body Fluids During Exercise 30th Annual International IEEE EMBS Conference Vancouver, British Columbia, Canada, August 20-24, 2008, and Novel lactate and pH biosensor for skin and sweat analysis based on single walled carbon nanotubes/Sensors and Actuators B 117 (2006) 308-313) .

However, an enzyme used in such a method is generally expensive and is susceptible to temperature, humidity, etc., and therefore has problems that stable properties are hard to exhibit and the reliability of quantitativeness is low. In addition, the enzyme greatly varies in quality among production lots or manufacturers, and also has a problem that its properties change greatly over time.

### SUMMARY

An advantage of some aspects of the invention is to provide a sensor capable of easily and stably performing detection of the strength of a stimulus (the concentration or the like of a given component) in a wide range, and also to provide a gel for a sensor which can be favorably used for a sensor capable of easily and stably performing detection of the strength of a stimulus (the concentration or the like of a given component) in a wide range.

A gel for a sensor according to an aspect of the invention includes a stimulus-responsive gel which reversibly expands or contracts in response to a given stimulus, and an electrically conductive gel containing electrically conductive particles constituted by a material containing an electrically conductive substance.

According to this configuration, a gel for a sensor which can be favorably used for a sensor capable of easily and stably performing detection of the strength of a stimulus (the concentration or the like of a given component) in a wide range can be provided.

In the gel for a sensor according to the aspect of the invention, it is preferred that the average particle diameter of the electrically conductive particles is 10 nm or more and 1000 µm or less.

In the gel for a sensor according to the aspect of the invention, it is preferred that the electrical resistivity of the electrically conductive substance is 1.0×10⁻³ Ω·m or less.

In the gel for a sensor according to the aspect of the invention, it is preferred that the electrically conductive substance is constituted by a material containing one or more members selected from the group consisting of a metal material, an electrically conductive metal oxide, a carbon material, and an electrically conductive polymer material.

In the gel for a sensor according to the aspect of the invention, it is preferred that the electrically conductive gel is provided with a recessed portion in a region coming into contact with an electrode.

In the gel for a sensor according to the aspect of the invention, it is preferred that in the recessed portion, the electrically conductive particles are exposed on the surface.

A sensor according to an aspect of the invention includes the gel for a sensor according to the aspect of the invention and an electrode connected to the electrically conductive gel.

According to this configuration, a sensor capable of easily and stably performing detection of the strength of a stimulus (the concentration or the like of a given component) in a wide range can be provided.

A sensor according to an aspect of the invention includes a stimulus-responsive gel which reversibly expands or contracts in response to a given stimulus, an electrically conductive gel containing electrically conductive particles constituted by a material containing an electrically conductive substance, and an electrode connected to the electrically conductive gel.

According to this configuration, a sensor capable of easily and stably performing detection of the strength of a stimulus (the concentration or the like of a given component) in a wide range can be provided.

It is preferred that the sensor according to the aspect of the invention further includes a housing member having a housing portion in which the stimulus-responsive gel and the electrically conductive gel are housed.

In the sensor according to the aspect of the invention, it is preferred that the housing member is constituted by a material which is harder than the stimulus-responsive gel and the electrically conductive gel.

In the sensor according to the aspect of the invention, it is preferred that the housing member is provided with an inflow port for allowing a specimen to flow in the housing portion.

In the sensor according to the aspect of the invention, it is preferred that when the stimulus-responsive gel expands, a pressing force acts on the electrically conductive gel in the stacking direction of the stimulus-responsive gel and the electrically conductive gel.

In the sensor according to the aspect of the invention, it is preferred that the stimulus-responsive gel is configured so that when the stimulus-responsive gel is deformed from a contracted state to an expanded state, the percentage of deformation thereof toward the direction in which the electrically conductive gel is provided is larger than the percentage of deformation thereof toward the other directions.

In the sensor according to the aspect of the invention, it is preferred that the sensor is used in a state where the stimulus-responsive gel is disposed on the side where a specimen is supplied closer than the electrically conductive gel.

In the sensor according to the aspect of the invention, it is preferred that the electrode is disposed on the side of the face of the electrically conductive gel opposite to the face on the side facing the stimulus-responsive gel.

In the sensor according to the aspect of the invention, it is preferred that the width of the face of the stimulus-responsive gel facing the electrically conductive gel is smaller than the length in the normal direction of the face.

In the sensor according to the aspect of the invention, it is preferred that the stimulus-responsive gel has a portion with a tapered cross-sectional area, in which the cross-sectional area of the portion is gradually reduced from the face on the side where the specimen is supplied to the face on the opposite side.

In the sensor according to the aspect of the invention, it is preferred that when a region where the stimulus-responsive gel and the electrically conductive gel are in contact with each other is seen in a plan view from the normal direction of the contact face of the electrically conductive gel with the stimulus-responsive gel, the region is overlapped with a range including at least a portion of a region between the electrodes.

### BRIEF DESCRIPTION OF THE DRAWINGS

The invention will be described with reference to the accompanying drawings, wherein like numbers reference like elements.

FIGS. 1A and 1B are schematic longitudinal cross-sectional views for illustrating a sensor according to a first embodiment, and FIG. 1A is a view showing a stimulus-responsive gel in a contracted state, and FIG. 1B is a view showing the stimulus-responsive gel in an expanded state.

FIG. 2 is a schematic plan view for illustrating a positional relationship among a stimulus-responsive gel, an electrically conductive gel, and electrodes in the sensor according to the first embodiment.

FIGS. 3A and 3B are schematic longitudinal cross-sectional views for illustrating a sensor according to a second embodiment, and FIG. 3A is a view showing a stimulus-responsive gel in a contracted state, and FIG. 3B is a view showing the stimulus-responsive gel in an expanded state.

### DESCRIPTION OF EXEMPLARY EMBODIMENTS

Hereinafter, preferred embodiments will be described in detail with reference to the accompanying drawings.

### Sensor and Gel for Sensor

Hereinafter, a sensor (gel sensor) and a gel for a sensor will be described.

### First Embodiment

First, a sensor according to a first embodiment and a gel for a sensor will be described.

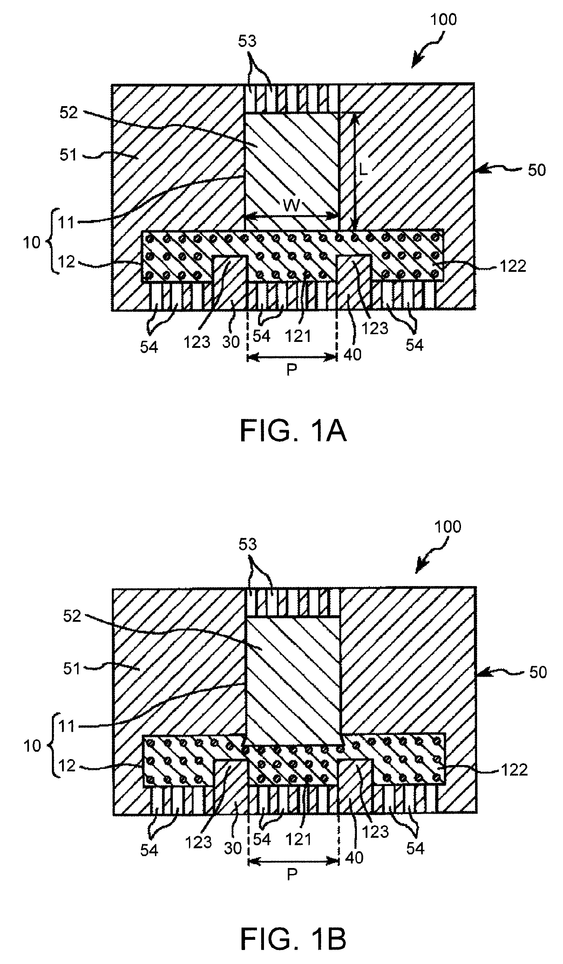

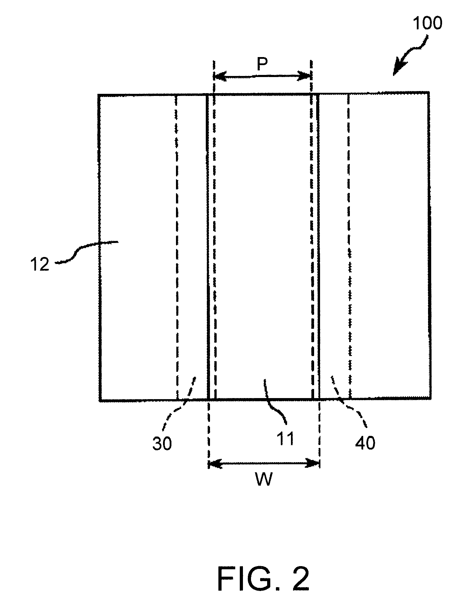

FIGS. 1A and 1B are schematic longitudinal cross-sectional views for illustrating a sensor according to a first embodiment, and FIG. 1A is a view showing a stimulus-responsive gel in a contracted state, and FIG. 1B is a view showing the stimulus-responsive gel in an expanded state. FIG. 2 is a schematic plan view for illustrating a positional relationship among the stimulus-responsive gel, an electrically conductive gel, and electrodes in the sensor according to the first embodiment. In the following description, a case where the upper side in FIGS. 1A and 1B is a side where a specimen is supplied will be mainly described.

As shown in FIGS. 1A and 1B, a sensor (gel sensor) 100 includes a gel for a sensor 10, a first electrode (electrode) 30, a second electrode (electrode) 40, and a housing member 50 in which the gel for a sensor 10 is housed. Then, the gel for a sensor 10 includes a stimulus-responsive gel 11, which expands or contracts in response to a given stimulus, and an electrically conductive gel 12 containing a plurality of electrically conductive particles 121 constituted by a material containing an electrically conductive substance.

According to such a configuration, the electrically conductive gel 12 is deformed according to the expanded state or the contracted state of the stimulus-responsive gel 11. As a result, a distance between the electrically conductive particles 121 contained in the electrically conductive gel 12 can be changed, so that a resistance value between the first electrode 30 and the second electrode 40 can be made different. That is, when the stimulus-responsive gel 11 (gel for a sensor 10) is in an expanded state (or in a state where the degree of expansion is large), the distance between the electrically conductive particles 121 is larger than when the stimulus-responsive gel 11 is in a contracted state (or in a state where the degree of expansion is small), and therefore, the resistance value of the gel for a sensor 10 connected to the electrodes (the first electrode 30 and the second electrode 40) is increased. Accordingly, by measuring the resistance value between the first electrode 30 and the second electrode 40, whether the stimulus-responsive gel 11 is in an expanded state or in a contracted state, that is, the strength of a stimulus (the concentration or the like of a given component) can be easily and stably found. In particular, the sensor (gel sensor) 100 is capable of easily and stably performing detection of the strength of a stimulus in a wide range.

### Stimulus-Responsive Gel

The stimulus-responsive gel 11 reversibly expands or contracts in response to a given stimulus.

In this manner, since the stimulus-responsive gel 11 is a material that responds to a given stimulus, also the electrically conductive gel 12 provided in contact with the stimulus-responsive gel 11 is deformed according to the response (expansion or contraction) of the stimulus-responsive gel 11, and therefore, the resistance value of the gel for a sensor 10 connected to the electrodes (the first electrode 30 and the second electrode 40) can be changed. As a result, by measuring the resistance value, the presence or absence of the stimulus, the strength (amount or concentration) thereof, etc. can be detected.

The given stimulus to which the stimulus-responsive gel 11 responds varies depending on the constituent material or the like of the stimulus-responsive gel 11, however, examples thereof include various types of substances such as proteins, sugars, uric acid, lactic acid, various types of hormones, various types of ionic substances, and various types of metals, heat, and light.

The constituent material of the stimulus-responsive gel 11 will be described in detail later.

The volume of the stimulus-responsive gel 11 included in the sensor 100 is preferably 0.1 mm³ or more and 3600 mm³ or less, more preferably 0.2 mm³ or more and 900 mm³ or less.

According to this, while reducing the size of the sensor 100, the stimulus detection accuracy can be made more excellent. Further, from the viewpoint of resource saving, the above configuration is preferred.

In this embodiment, the stimulus-responsive gel 11 is disposed on the side where a specimen is supplied closer than (on the upstream side of) the electrically conductive gel 12.

According to this, a time from when the specimen comes into contact with the gel for a sensor 10 to when the stimulus-responsive gel 11 is deformed and the stimulus is electrically detected can be shortened. In addition, an undesirable diffusion of the specimen in the gel for a sensor 10 or the like can be prevented, and thus, the specimen detection accuracy can be made more excellent.

In the following description, the face of the stimulus-responsive gel 11 on the side where a specimen is supplied (the face on the upper side in FIGS. 1A and 1B) is referred to as "first face 111", and the face of the stimulus-responsive gel 11 on the side opposite to the first face 111 (the face on the side facing the electrically conductive gel 12) is referred to as "second face 112".

In this embodiment, the width W of the face (second face) 112 of the stimulus-responsive gel 11 on the side facing the electrically conductive gel 12 (on the lower side in FIGS. 1A and 1B) is smaller than the length L in the normal direction of the face 112.

According to this, the effect of deformation of the stimulus-responsive gel 11 in response to a given stimulus can be more efficiently given to the electrically conductive gel 12, and thus, the stimulus detection accuracy can be made more excellent.

Such a relationship is preferably satisfied at least when the stimulus-responsive gel 11 is in a contracted state (see FIG. 1A), and is more preferably satisfied also when the stimulus-responsive gel 11 is in an expanded state (see FIG. 1B) in addition to when the stimulus-responsive gel 11 is in a contracted state.

The thickness (length L) of the stimulus-responsive gel 11 in a contracted state is not particularly limited, but is preferably 5 µm or more and 5000 µm or less, more preferably 7 µm or more and 1000 µm or less.

According to this, while making the durability and reliability of the sensor 100 sufficiently excellent, the thickness and size of the sensor 100 can be reduced. Further, a pressing force of the stimulus-responsive gel 11 against the electrically conductive gel 12 when the stimulus-responsive gel 11 is transformed from the contracted state to the expanded state can be made relatively large, and thus, the degree of deformation of the electrically conductive gel 12 can be made larger while preventing the breakage of the electrically conductive gel 12, and therefore, the stimulus detection accuracy can be made more excellent. In addition, the flexibility of the sensor 100 can be made more excellent, and for example, even in the case where the sensor 100 is used in close contact with a living body or the like, the adhesiveness of the sensor 100 can be favorably maintained, and the stimulus detection accuracy can be stably made excellent.

### Electrically Conductive Gel

The electrically conductive gel 12 is configured such that the electrically conductive particles 121 constituted by a material containing an electrically conductive substance are dispersed in a gel material 122.

In this embodiment, the electrically conductive gel 12 is disposed in contact with the stimulus-responsive gel 11.

According to this, the effect of deformation of the stimulus-responsive gel 11 in response to a given stimulus can be more efficiently given to the electrically conductive gel 12, and thus, the stimulus detection accuracy can be made more excellent.

In particular, in this embodiment, the electrically conductive gel 12 is in contact with the second face 112 which is the face on the side opposite to the first face (face) 111 of the stimulus-responsive gel 11.

According to this, the effect of deformation of the stimulus-responsive gel 11 in response to a given stimulus can be more efficiently given to the electrically conductive gel 12 without disturbing the contact between a specimen and the stimulus-responsive gel 11, and thus, the stimulus detection accuracy can be made more excellent.

### Electrically Conductive Particles

In the electrically conductive gel 12, a plurality of electrically conductive particles 121 constituted by a material containing an electrically conductive substance are contained.

According to this, a distance between the electrically conductive particles 121 changes according to the deformation of the electrically conductive gel 12 accompanying the response (expansion or contraction) of the stimulus-responsive gel 11 to a given stimulus, and the resistance value of the gel for a sensor 10 connected to the electrodes (the first electrode 30 and the second electrode 40) can be changed. As a result, by measuring the resistance value, the presence or absence of the stimulus, the strength (amount or concentration) thereof, etc. can be detected.

Examples of the electrically conductive substance constituting the electrically conductive particles 121 include metal materials (for example, Au, Ag, Pt, Cu, an alloy containing at least one member selected from these, and the like), electrically conductive metal oxides (for example, ITO and the like), carbon materials (for example, carbon black and the like), and electrically conductive polymer materials (for example, PEDOT/PSS, a polythiophene-based material, a polyacetylene-based material, and the like).

By including the electrically conductive particles 121 constituted by such a material, while making the durability of the sensor 100 excellent, the percentage of change in the resistance value accompanying the expansion or contraction of the stimulus-responsive gel 11 can be further increased, and thus, the stimulus detection accuracy can be made more excellent. In addition, these materials are relatively inexpensive and can be stably obtained, and therefore are advantageous also from the viewpoint of suppression of the production cost of the sensor 100 and stable supply thereof.

The electrical resistivity of the electrically conductive substance constituting the electrically conductive particles 121 is preferably 1.0×10⁻³ Ω·m or less, more preferably 1.0×10⁻⁴ Ω·m or less, further more preferably 5.0 ×10⁻⁵ Ω·m or less.

According to this, the percentage of change in the resistance value accompanying the expansion or contraction of the stimulus-responsive gel 11 can be further increased, and thus, the stimulus detection accuracy can be made more excellent.

The electrically conductive particle 121 may have a composition uniform in all regions, or may have a region having a different composition.

For example, the electrically conductive particle 121 may be a particle which has a core portion and at least one coating layer provided on the outer surface side of the core portion, or the like.

Further, the electrically conductive particle 121 may be a particle subjected to a surface treatment for improving the dispersibility in the gel material 122.

According to this, an undesirable variation in the composition among the respective regions of the electrically conductive gel 12 connected to the electrodes (the first electrode 30 and the second electrode 40) can be suppressed, and thus, the stability of the stimulus detection accuracy can be made more excellent.

Examples of a surface treatment agent which can be used in the surface treatment include agents having a hydrophobic functional group such as an octadecyl group, a hydrophilic functional group such as a hydroxy group, a carboxyl group, or a sulfonic acid group (sulfo group), a salt thereof, or the like in a partial structure. The type of the surface treatment agent can be selected according to, for example, the type or the like of a solvent constituting the gel material 122.

As described above, in the case where the electrically conductive particle 121 has a region having a different composition, the electrically conductive particle 121 may have electrical conductivity to such an extent that the effect as described above can be exhibited as a whole, and a portion of the electrically conductive particle 121 may be constituted by an insulating material. For example, in the case where at least a portion of the base particle of the electrically conductive particle 121 is constituted by a material having electrical conductivity, a portion formed by the surface treatment (a surface-treated portion) may be constituted by an insulating material.

The electrically conductive particle 121 may have any shape such as a spherical shape, a spindle shape, a needle shape (a spiny shape), a plate shape (a scale shape), or a disk shape such as an erythrocyte shape, but is preferably has a spherical shape.

According to this, the reliability of the electrical resistivity to be detected is further enhanced.

In addition, the electrically conductive particle 121 may exist in the gel for a sensor 10 as an aggregate formed by aggregating a plurality of particles.

According to this, the electrical conductivity of the gel for a sensor 10 is easily ensured, and thus, the detection of a stimulus can be more favorably performed.

The average particle diameter of the electrically conductive particles 121 is not particularly limited, but is preferably 10 nm or more and 1000 µm or less, more preferably 20 nm or more and 500 µm or less.

According to this, in the electrically conductive gel 12, the electrically conductive particles 121 can be more uniformly dispersed, and the stimulus detection accuracy can be made more excellent.

The "average particle diameter" as used herein refers to an average particle diameter on the volume basis, and can be obtained by, for example, performing measurement using a particle size distribution analyzer employing a Coulter counter method (model: TA-II, manufactured by Coulter Electronics, Inc.) with an aperture of 50 µm for a dispersion liquid obtained by adding a sample to methanol and dispersing the sample therein for 3 minutes with an ultrasonic disperser.

The gel for a sensor 10 may contain a plurality of types of electrically conductive particles 121.

The content of the electrically conductive particles 121 with respect to 100 parts by volume of the electrically conductive gel 12 is preferably 0.1 parts by volume or more and 50 parts by volume or less, more preferably 0.5 parts by volume or more and 40 parts by volume or less.

According to this, the amount of change in the electrical resistivity depending on the state (the expanded state or the contracted state) of the stimulus-responsive gel 11 can be further increased, and thus, the stimulus detection accuracy and detection sensitivity can be made more excellent.

### Gel Material

The gel material 122 constituting the electrically conductive gel 12 has a function to disperse the above-mentioned electrically conductive particles 121.

As the gel material 122, for example, a material containing a gelling agent and a solvent can be favorably used.

As the gelling agent, for example, various synthetic polymer materials such as polyacrylamide, polydimethylacrylamide, a carboxyvinyl polymer, and a vinylidene fluoride-hexafluoride propylene copolymer, natural polymer materials such as agar, gelatin, carrageenan, pectin, an alginate, gellan gum, and glucomannan, and other than these, methyl (meth)acrylate, an organic electrolyte oligomer, and the like can be used. Further, a semi-synthetic product obtained by subjecting a natural polymer material to chemical modification (including cleavage of a molecular chain and a polymerization reaction) can also be used. As the gelling agent, a polymer material as the constituent component of the stimulus-responsive gel 11 as described in detail later can also be preferably used. According to this, the detection accuracy and the detection sensitivity for a given stimulus can be made more excellent.

The electrical resistivity of the gel material 122 alone (the electrical resistivity of a material in which the electrically conductive particles 121 are not dispersed) is preferably 1.0×10⁻² Ω·m or more, more preferably 1.0×10⁻¹ Ω·m or more.

According to this, the difference in the electrical resistivity of the electrically conductive gel 12 between the contracted state and the expanded state of the stimulus-responsive gel 11 can be made larger, and the stimulus detection accuracy can be made more excellent.

The sensor 100 is configured such that when the stimulus-responsive gel 11 expands, a pressing force acts on the electrically conductive gel 12 in the stacking direction of the stimulus-responsive gel 11 and the electrically conductive gel 12 (the vertical direction in FIGS. 1A and 1B) .

According to this, the effect of deformation of the stimulus-responsive gel 11 in response to a given stimulus can be more efficiently given to the electrically conductive gel 12, and thus, the stimulus detection accuracy can be made more excellent.

As shown in FIG. 2, in this embodiment, when a region where the stimulus-responsive gel 11 and the electrically conductive gel 12 are in contact with each other is seen in a plan view from the normal direction (the upper side in FIGS. 1A and 1B) of the contact face of the electrically conductive gel 12 with the stimulus-responsive gel 11, the region is overlapped with a range including a region between the electrodes 30 and 40.

According to this, the effect of deformation of the stimulus-responsive gel 11 in response to a given stimulus can be more efficiently given to the electrically conductive gel 12, and thus, the stimulus detection accuracy can be made more excellent.

The difference between the electrical resistivity of the electrically conductive gel 12 when the stimulus-responsive gel 11 is in an expanded state (the maximum electrical resistivity) and the electrical resistivity of the electrically conductive gel 12 when the stimulus-responsive gel 11 is in a contracted state (the minimum electrical resistivity) is preferably 1.0×10⁻⁵ Ω·m or more, more preferably 1.0×10⁻² Ω·m or more.

According to this, the stimulus detection accuracy can be made more excellent.

In this embodiment, a recessed portion 123 is provided in regions coming into contact with the electrodes 30 and 40 of the electrically conductive gel 12.

According to this, the positioning of the electrically conductive gel 12 (the gel for a sensor 10) and the electrodes 30 and 40 is facilitated, and thus, the stability of detection of a stimulus and the reliability of the sensor 100 can be made more excellent.

The electrically conductive gel 12 may contain a component other than the above-mentioned components.

For example, the electrically conductive gel 12 may contain particles (non-electrically conductive particles) other than the electrically conductive particles 121.

### Electrode

To the electrically conductive gel 12, the first electrode (electrode) 30 and the second electrode (electrode) 40 are connected.

According to this, the resistance value of the gel for a sensor 10 can be favorably measured, and from this measurement result, the presence or absence of a given stimulus received by the stimulus-responsive gel 11 or the strength thereof can be determined.

In particular, in this embodiment, the electrodes 30 and 40 are disposed on the side of the face of the electrically conductive gel 12 opposite to the face on the side facing the stimulus-responsive gel 11.

According to this, the measurement of the resistance value can be easily performed, and also favorable adhesiveness between the electrodes 30 and 40 and the electrically conductive gel 12 can be more favorably maintained.

Further, in this embodiment, the electrodes 30 and 40 are provided on the face of the gel for a sensor 10 on the side opposite to the side where the specimen is supplied.

According to this, for example, even in the case where the specimen is a liquid having electrical conductivity such as sweat, the occurrence of a short circuit due to the specimen existing outside the gel for a sensor 10 can be more effectively prevented, and thus, the reliability of the detection of the stimulus can be made more excellent.

In the configuration shown in the drawing, the electrodes 30 and 40 are provided in contact with the electrically conductive particles 121 exposed on the surface of the electrically conductive gel 12 (the surface in the recessed portion 123).

According to this, the stability of detection of a stimulus and the reliability of the sensor 100 can be made more excellent.

The electrodes 30 and 40 are constituted by a material having electrical conductivity.

Examples of the constituent material of the electrodes 30 and 40 include metal materials (for example, Au, Ag, Pt, Cu, an alloy containing at least one member selected from these, and the like), electrically conductive metal oxides (for example, ITO and the like), carbon materials (for example, carbon black and the like), and electrically conductive polymer materials (for example, PEDOT/PSS, a polythiophene-based material, a polyacetylene-based material, and the like).

The thickness of the electrodes 30 and 40 is not particularly limited, but is preferably 5 µm or more and 5000 µm or less, more preferably 7 µm or more and 1000 µm or less.

According to this, while making the durability and reliability of the sensor 100 sufficiently excellent, the thickness and size of the sensor 100 can be reduced. In addition, the flexibility of the sensor 100 can be made more excellent, and for example, even in the case where the sensor 100 is used in close contact with a living body or the like, the adhesiveness of the sensor 100 can be favorably maintained, and the stimulus detection accuracy can be stably made excellent.

A distance between the electrode 30 and the electrode 40 denoted by P in the drawing (inter-electrode distance) is preferably 0.1 mm or more and 50 mm or less, more preferably 0.2 mm or more and 30 mm or less.

According to this, while suppressing the increase in the size of the sensor 100, the reliability of the detection of a stimulus can be made more excellent.

### Housing Member

The housing member 50 has a function to house the gel for a sensor 10, and includes a tangible portion 51 which protects the gel for a sensor 10 and a housing portion 52 which houses the gel for a sensor 10.

By including such a housing member 50, the gel for a sensor 10 can be favorably protected and the detection of a stimulus can be stably performed.

In this embodiment, by the tangible portion 51 of the housing member 50, the expansion of the stimulus-responsive gel 11 to a direction perpendicular to the stacking direction of the stimulus-responsive gel 11 and the electrically conductive gel 12 (the vertical direction in FIGS. 1A and 1B) is regulated, and the stimulus-responsive gel 11 is configured so that when the stimulus-responsive gel 11 is deformed from the contracted state to the expanded state, the amount of deformation thereof toward the direction in which the electrically conductive gel 12 is provided is larger than the amount of deformation thereof toward the other directions.

According to this, a pressing force applied to the electrically conductive gel 12 can be more favorably adjusted according to the strength of a stimulus (the amount of a stimulus or the strength of a stimulus) received by the stimulus-responsive gel 11, and thus, the stimulus detection accuracy can be made more excellent.

In particular, in the configuration shown in the drawing, when the stimulus-responsive gel 11 is in a contracted state (see FIG. 1A), the side face (the face in a direction perpendicular to the face facing the electrically conductive gel 12) of the stimulus-responsive gel 11 is in contact with the tangible portion 51 of the housing member 50.

According to this, the effect as described above is more remarkably exhibited.

The tangible portion 51 of the housing member 50 is preferably constituted by a material which is harder than the stimulus-responsive gel 11 and the electrically conductive gel 12.

According to this, the cell for a sensor 10 can be favorably protected, and the detection of a stimulus can be more stably performed, and also the effect of deformation of the stimulus-responsive gel 11 in response to a given stimulus can be more efficiently given to the electrically conductive gel 12, and thus, the stimulus detection accuracy can be made more excellent.

Examples of the constituent material of the tangible portion 51 include polyolefins such as polyethylene, polypropylene, polybutadiene, and ethylene-vinyl acetate copolymers; polyesters such as polyvinyl chloride, polyurethane, polystyrene, polycarbonate, polyamide, polyethylene terephthalate, and polybutylene terephthalate; acrylic resins such as polymethyl methacrylate; ABS resins, AS resins, ionomers, polyacetal, polyphenylene sulfide, polyether ether ketone, various rubber materials such as natural rubber, butyl rubber, isoprene rubber, butadiene rubber, styrene-butadiene rubber, silicone rubber, and fluororubber; silicone-based materials such as dimethylpolysiloxane; and various thermoplastic elastomers such as polyurethane-based, polyester-based, polyamide-based, olefin-based, and styrene-based thermoplastic elastomers.

The housing member 50 is provided with an inflow port (opening) 53 for allowing a specimen which is a fluid (for example, sweat or the like) to flow in the housing portion 52.

According to this, while reliably protecting the gel for a sensor 10, the gel for a sensor 10 (stimulus-responsive gel 11) and the specimen can be more favorably brought into contact with each other, and therefore, the detection of a given stimulus can be performed at a higher speed, and also the detection accuracy for a given stimulus can be made more excellent.

The inflow port 53 may be provided at any place, however, in the configuration shown in the drawing, it is provided at a place facing the stimulus-responsive gel 11 in the gel for a sensor 10.

According to this, the specimen can be more efficiently supplied to the stimulus-responsive gel 11, and also the effect as described above is more remarkably exhibited.

The shape of the inflow port 53 may be any shape, and examples thereof include a slit shape and a circle shape.

Further, the inflow port 53 may be, for example, a space among fibers constituting a woven fabric or a non-woven fabric, or may be a pore of a porous material.

Further, the housing member 50 is provided with a discharge port (opening) 54 for discharging a specimen which is a fluid from the housing portion 52.

According to this, for example, in the case where a specimen is sequentially supplied (in the case where it is supplied continuously or intermittently), the previously supplied specimen is discharged, and the newly supplied specimen can be supplied to the stimulus-responsive gel 11, and therefore, a change in the amount of the stimulus over time can be found. That is, the detection of the accurate amount of the stimulus can be prevented from being disturbed due to mixing of the previously supplied specimen with the newly supplied specimen. In addition, even in the case where an excess amount of a specimen is supplied or the supply rate of a specimen is increased, it is possible to more effectively prevent undesirable deformation by applying a large pressure to the stimulus-responsive gel 11 due to an excess pressure generated by the supplied specimen.

The discharge port 54 may be provided at any place, however, in the configuration shown in the drawing, it is provided at a place facing the electrically conductive gel 12 in the gel for a sensor 10.

According to this, the effect as described above is more remarkably exhibited.

The shape of the discharge port 54 may be any shape, and examples thereof include a slit shape and a circle shape.

Further, the discharge port 54 may be, for example, a space among fibers constituting a woven fabric or a non-woven fabric, or may be a pore of a porous material.

In addition, the sensor 100 may include an absorbing member (not shown) which absorbs a specimen.

According to this, for example, in the case where an excess amount of a specimen exists outside the gel for a sensor 10, the excess amount of the specimen can be absorbed by the absorbing member, and even in the case where the specimen is a liquid having electrical conductivity such as sweat, the occurrence of a short circuit can be more effectively prevented, and thus, the reliability of the detection of the stimulus can be made more excellent. Further, in the case where a specimen is sequentially supplied (in the case where it is supplied continuously or intermittently), the previously supplied specimen is discharged, and the newly supplied specimen can be supplied to the stimulus-responsive gel 11, and therefore, a change in the amount of the stimulus over time can be found. That is, the detection of the accurate amount of the stimulus can be prevented from being disturbed due to mixing of the previously supplied specimen with the newly supplied specimen. In addition, for example, in the case where the supply of a liquid specimen is stopped or in the case where the supply amount of a specimen is significantly decreased, when the use environment of the sensor 100 is a low humidity environment or the like, the stimulus-responsive gel 11 can be prevented from being dried. As a result, even in an environment in which the stimulus-responsive gel 11 is easily dried, the detection of a given stimulus can be stably performed with high reliability over a relatively long period of time.

The absorbing member may be disposed in any place, but is preferably disposed in a place different from the face of the gel for a sensor 10 on the side where the specimen is supplied.

According to this, while more favorably preventing the supply of the specimen to the stimulus-responsive gel 11 from being inhibited, the effect as described above can be exhibited.

In particular, the absorbing member is preferably disposed on the face of the gel for a sensor 10 on the side opposite to the side where the specimen is supplied (on the side of the face on which the electrically conductive gel 12 is disposed).

According to this, the effect as described above is more remarkably exhibited, and in particular, in the case where the specimen is sequentially supplied (in the case where it is supplied continuously or intermittently), the previously supplied specimen is discharged, and the newly supplied specimen can be favorably supplied to the stimulus-responsive gel 11, and therefore, a change in the amount of the stimulus over time can be favorably found.

In addition, the absorbing member is preferably disposed so as not to come into contact with a plurality of electrodes.

According to this, while exhibiting the effect as described above, for example, even in the case where the specimen is a liquid having electrical conductivity such as sweat, the occurrence of a short circuit due to the specimen existing outside the gel for a sensor 10 can be more effectively prevented, and thus, the reliability of the detection of the stimulus can be made more excellent.

Examples of the absorbing member include members constituted by a woven fabric, a non-woven fabric, a cloth-like material such as felt, a porous material, or the like.

As a preferred constituent component of the absorbing member, a cellulose material, a water-absorbing polymer, or the like can be used, however, a cellulose material is particularly preferred. Such a material has moderate hydrophilicity, and therefore, for example, in the case where the specimen contains water (for example, a body fluid such as sweat), the specimen can be favorably absorbed in the material, and also water contained in the absorbed specimen can be favorably evaporated from the material.

### Second Embodiment

Next, a sensor according to a second embodiment will be described.

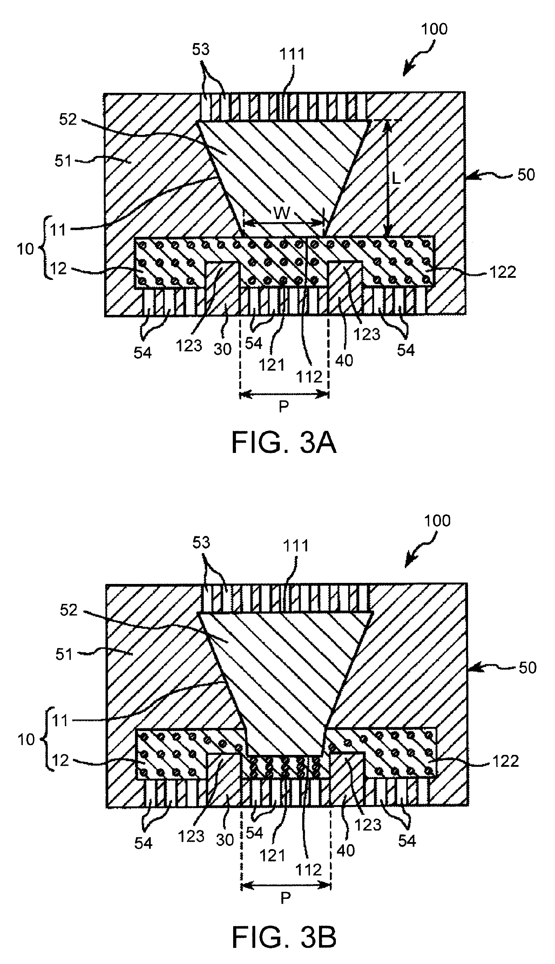

FIGS. 3A and 3B are schematic longitudinal cross-sectional views for illustrating a sensor according to a second embodiment, and FIG. 3A is a view showing a stimulus-responsive gel in a contracted state, and FIG. 3B is a view showing the stimulus-responsive gel in an expanded state. In the following description, different points from the above-mentioned embodiment will be mainly described, and the description of the same matter will be omitted.

The sensor (gel sensor) 100 of this embodiment has the same configuration as that of the above-mentioned embodiment except that the shape of the electrically conductive gel 12 and the shape of the housing portion 52 which houses the gel for a sensor 10. That is, in the above-mentioned embodiment, the stimulus-responsive gel 11 is in the form of a rectangular parallelepiped, and the cross-sectional shape of the stimulus-responsive gel 11 is a rectangle, however, in the sensor (gel sensor) 100 of this embodiment, the stimulus-responsive gel 11 has a portion with a tapered cross-sectional area, in which the cross-sectional area of the portion is gradually reduced from the face on the side where a specimen is supplied to the face on the opposite side (from the upper side to the lower side in FIGS. 3A and 3B).

According to this, the amount of a specimen with which the stimulus-responsive gel 11 comes into contact can be increased, and also the effect of deformation of the stimulus-responsive gel 11 in response to a given stimulus can be more efficiently given to the electrically conductive gel 12, and thus, the stimulus detection accuracy and detection sensitivity can be made more excellent.

### Constituent Material of Stimulus-Responsive Gel

Next, the constituent material of the stimulus-responsive gel constituting the sensor will be described.

The stimulus-responsive gel may be constituted by any material as long as it responds to a given stimulus, but is generally constituted by a material containing a polymer material having a crosslinked structure and a solvent.

### Polymer Material

The polymer material constituting the stimulus-responsive gel is an important component for detecting a specific stimulus, and the structure thereof varies depending on the type of the stimulus to be detected.

The polymer material constituting the stimulus-responsive gel is not particularly limited, and can be selected according to the stimulus to be detected.

Hereinafter, specific examples of the polymer material constituting the stimulus-responsive gel will be described.

As the polymer material constituting the stimulus-responsive gel, for example, a polymer material obtained by reacting a monomer, a polymerization initiator, a crosslinking agent, etc. can be used.

Examples of the monomer include acrylamide, N-methylacrylamide, N-isopropylacrylamide, N,N-dimethylacrylamide, N,N-dimethylaminopropylacrylamide, various quaternary salts of N,N-dimethylaminopropylacrylamide, acryloylmorpholine, various quaternary salts of N,N-dimethylaminoethylacrylate, acrylic acid, various alkyl acrylates, methacrylic acid, various alkyl methacrylates, 2-hydroxyethylmethacrylate, glycerol monomethacrylate, N-vinylpyrrolidone, acrylonitrile, styrene, polyethylene glycol diacrylate, 1,6-hexanediol diacrylate, neopentyl glycol diacrylate, tripropylene glycol diacrylate, polypropylene glycol diacrylate, 2,2-bis[4-(acryloxydiethoxy)phenyl]propane, 2,2-bis[4-(acryloxypolyethoxy)phenyl]propane, 2-hydroxy-1-acryloxy-3-methacryloxypropane, 2,2-bis[4-(acryloxypolypropoxy)phenyl]propane, ethylene glycol dimethacrylate, diethylene glycol dimethacrylate, triethylene glycol dimethacrylate, polyethylene glycol dimethacrylate, 1,3-butylene glycol dimethacrylate, 1,6-hexanediol dimethacrylate, neopentyl glycol dimethacrylate, polypropylene glycol dimethacrylate, 2-hydroxy-1,3-dimethacryloxypropane, 2,2-bis[4-(methacryloxyethoxy)phenyl]propane, 2,2-bis[4-(methacryloxyethoxydiethoxy)phenyl]propane, 2,2-bis[4-(methacryloxyethoxypolyethoxy)phenyl]propane, trimethylolpropane trimethacrylate, tetramethylolmethane trimethacrylate, trimethylolpropane triacrylate, tetramethylolmethane triacrylate, tetramethylolmethane tetraacrylate, dipentaerythritol hexaacrylate, N,N'-methylenebisacrylamide, N,N'-methylenebismethacrylamide, diethylene glycol diallyl ether, and divinylbenzene.

Further, examples of a functional group which can interact with a sugar include a boronic acid group (particularly, a phenylboronic acid group), and therefore, a monomer having a boronic acid group may be used. Examples of such a boronic acid group-containing monomer include acryloylaminobenzeneboronic acid, methacryloylaminobenzeneboronic acid, and 4-vinylbenzeneboronic acid.

In the case where an ionic substance (particularly, an ionic substance containing a calcium ion) is detected as a stimulus (specific component), a crown ether group-containing monomer (particularly, a benzocrown ether group-containing monomer) such as 4-acrylamidobenzo-18-crown 6-ether, acryloyl aminobenzocrown ether, methacryloyl aminobenzocrown ether, or 4-vinylbenzocrown ether can be preferably used as the monomer.

In the case where an ionic substance such as sodium chloride is detected as a stimulus (specific component), 3-acrylamidophenylboronic acid, vinylphenylboronic acid, acryloyloxyphenylboronic acid, N-isopropylacrylamide (NIPAAm), ethylenebisacrylamide, N-hydroxyethylacrylamide, or the like can be preferably used as the monomer. In particular, in the case where an ionic substance such as sodium chloride is detected as a stimulus (specific component), it is preferred to use at least one monomer selected from the group consisting of 3-acrylamidophenylboronic acid, vinylphenylboronic acid, and acryloyloxyphenylboronic acid, and at least one monomer selected from the group consisting of N-isopropylacrylamide (NIPAAm), ethylenebisacrylamide, and N-hydroxyethylacrylamide in combination as the monomer.

In the case where lactic acid is detected as a stimulus (specific component), 3-acrylamidophenylboronic acid, vinylphenylboronic acid, acryloyloxyphenylboronic acid, N-isopropylacrylamide (NIPAAm), ethylenebisacrylamide, N-hydroxyethylacrylamide, or the like can be preferably used as the monomer. In particular, in the case where lactic acid is detected as a stimulus (specific component), it is preferred to use at least one monomer selected from the group consisting of 3-acrylamidophenylboronic acid, vinylphenylboronic acid, and acryloyloxyphenylboronic acid, and at least one monomer selected from the group consisting of N-isopropylacrylamide (NIPAAm), ethylenebisacrylamide, and N-hydroxyethylacrylamide in combination as the monomer.

The polymerization initiator can be appropriately selected according to, for example, the polymerization method thereof. Specific examples thereof include compounds which generate radicals by ultraviolet light including hydrogen peroxide, persulfates such as potassium persulfate, sodium persulfate, and ammonium persulfate, azo-based initiators such as 2,2'-azobis(2-amidinopropane) dihydrochloride, 2,2'-azobis(N,N'-dimethyleneisobutylamidine) dihydrochloride, 2,2'-azobis{2-methyl-N-[1,1-bis(hydroxymethyl)-2-hydroxyet hyl]propionamide}, 2,2'-azobis[2-(2-imidazolin-2-yl)propane] dihydrochloride, 4,4'-azobis(4-cyanovaleric acid), 2,2'-azobisisobutyronitrile, 2,2'-azobis(2,4'-dimethylvaleronitrile), benzophenone, 2,2-dimethoxy-1,2-diphenylethan-1-one, 1-hydroxycyclohexyl phenyl ketone, 2-hydroxy-2-methyl-1-phenylpropan-1-one, 2,4,6-trimethylbenzoyl diphenylphosphine oxide, 1-[4-(2-hydroxyethoxy)-phenyl]-2-hydroxy-2-methyl-1-propan -1-one, and the like, and compounds which generate radicals by light with a wavelength of 360 nm or more such as substances obtained by mixing a thiopyrylium salt-based, merocyanine-based, quinoline-based, orstyrylquinoline-based dye with 2,4-diethyl thioxanthone, isopropyl thioxanthone, 1-chloro-4-propoxythioxanthone, 2-(3-dimethylamino-2-hydroxypropoxy)-3,4-dimethyl-9H-thiox anthon-9-one methochloride, 2-methyl-1[4-(methylthio)phenyl]-2-morpholinopropane-1,2-b enzyl-2-dimethylamino-1-(4-morpholinophenyl)-butanone-1, bis(cyclopentadienyl)-bis(2,6-difluoro-3-(pyl-1-yl) titanium, or a peroxy ester such as 1,3-di(t-butylperoxycarbonyl)benzene or 3,3',4,4'-tetra-(t-butylperoxycarbonyl)benzophenone. Hydrogen peroxide or a persulfate can also be used as a redox-based initiator in combination with, for example, a reducing substance such as a sulfite or L-ascorbic acid, an amine salt, or the like.

As the crosslinking agent, a compound having two or more polymerizable functional groups can be used, and specific examples thereof include ethylene glycol, propylene glycol, trimethylolpropane, glycerin, polyoxyethylene glycol, polyoxypropylene glycol, polyglycerin, N,N'-methylenebisacrylamide, N,N-methylene-bis-N-vinylacetamide, N,N-butylene-bis-N-vinylacetamide, tolylene diisocyanate, hexamethylene diisocyanate, allylated starch, allylated cellulose, diallyl phthalate, tetraallyloxyethane, pentaerythritol triallyl ether, trimethylolpropane triallyl ether, diethylene glycol diallyl ether, and triallyl trimellitate.

The stimulus-responsive gel may contain a plurality of different types of polymer materials.

For example, the stimulus-responsive gel may contain as the polymer material, a first polymer having an OH group (a hydroxy group which bonds to a carbon atom) and a second polymer having a phenylboronic acid structure.

According to this, the stimulus-responsive gel can be in a first state where the OH group possessed by the first polymer and the phenylboronic acid structure possessed by the second polymer are bonded to each other, and in a second state where the bond between the OH group possessed by the first polymer and the phenylboronic acid structure possessed by the second polymer is dissociated.

The change between these states occurs by a change in the surrounding environment (a change in the presence or absence of a given stimulus or a change in the strength or the like of a stimulus). Then, the ratio of molecules which are in the first state to molecules which are in the second state among the many molecules (the first polymer molecules and the second polymer molecules) contained in the stimulus-responsive gel changes with inclination according to the strength of the stimulus.

Due to this, the strength of the stimulus can be quantitatively detected.

It is preferred that the stimulus-responsive gel is transformed to the second state by reacting the phenylboronic acid structure possessed by the second polymer with lactic acid.

The stimulus-responsive gel containing the first polymer and the second polymer as described above can detect and quantitatively determine lactic acid among various stimuli with particularly high sensitivity in a particularly wide concentration range. In the past, the detection and quantitative determination of lactic acid were mostly performed using an enzyme, and there was no gel material capable of being favorably applied to the detection and quantitative determination of lactic acid. In view of this, by using the stimulus-responsive gel for the detection and quantitative determination of lactic acid, the effect of the invention is more remarkably exhibited.

The reason why the stimulus-responsive gel (the stimulus-responsive gel containing the first polymer and the second polymer) as described above shows high sensitivity to lactic acid is considered to be as follows. That is, in the case where the sensor is used for detecting lactic acid, when the concentration of lactic acid is low, the ratio of molecules in the first state where the OH group possessed by the first polymer and the phenylboronic acid structure possessed by the second polymer are bonded to each other is increased. On the other hand, when the concentration of lactic acid is increased, the bond between the OH group possessed by the first polymer and the phenylboronic acid structure possessed by the second polymer is replaced by a bond between lactic acid and the phenylboronic acid structure with extremely high reactivity. This is considered to be because lactic acid is a compound which has an α-hydroxycarboxylic acid structure and has particularly high reactivity with a phenylboronic acid structure, and also the size of the molecule is small, and therefore, in the stimulus-responsive gel, lactic acid can easily come close to the phenylboronic acid structure possessed by the second polymer. In addition, by bonding lactic acid to the phenylboronic acid structure constituting the second polymer, the hydrophilicity of the second polymer largely changes, and due to this, the expansion coefficient of the stimulus-responsive gel can be further increased. As a result, the stimulus (lactic acid) detection sensitivity and detection accuracy can be made more excellent.

Hereinafter, a case where the stimulus-responsive gel contains the first polymer having an OH group (a hydroxy group which bonds to a carbon atom) and the second polymer having a phenylboronic acid structure, in particular, a case where the stimulus-responsive gel is transformed to the second state by reacting the phenylboronic acid structure possessed by the second polymer with lactic acid and is used for the detection and quantitative determination of lactic acid will be mainly described.

### First Polymer

The first polymer has an OH group (hydroxy group).

The OH group may be an OH group introduced after polymerizing a polymerizable compound (a monomer or the like) as a constituent component of the polymer, but is preferably an OH group possessed by a polymerizable compound as a constituent component of the polymer.

According to this, the adjustment of the percentage or the like of the OH group possessed by the first polymer can be easily and reliably performed.

Examples of the monomer having an OH group which constitutes the first polymer include N-hydroxyethyl acrylamide, N,N'-(1,2-dihydroxyethylene)bisacrylamide, 2-hydroxyethyl methacrylate, glycerol monomethacrylate, 2-hydroxy-1-acryloxy-3-methacryloxypropane, and 2-hydroxy-1,3-dimethacryloxypropane, and it is possible to use one member or two or more members in combination selected from these, however, the first polymer preferably contains N-hydroxyethyl acrylamide as a constituent component.

According to this, the transformation between the first state and the second state according to the change in the environment (particularly the change in the concentration of lactic acid) in which the stimulus-responsive gel is placed is more favorably performed, and the detection and quantitative determination of the strength of a stimulus (particularly, the concentration of lactic acid) can be more stably performed in a wider range. In addition, the ability to retain the solvent of the stimulus-responsive gel can be made particularly excellent, and thus, a favorable gel state can be stably maintained over a long period of time.

The first polymer may contain a monomer having no OH group as a constituent component thereof. According to this, the percentage or the like of the OH group possessed by the first polymer can be adjusted to a favorable level.

Examples of the monomer having no OH group which constitutes the first polymer include acrylamide, N-methylacrylamide, N-isopropylacrylamide, N,N-dimethylacrylamide, N,N-dimethylaminopropyl acrylamide, various quaternary salts of N,N-dimethylaminopropyl acrylamide, acryloylmorpholine, various quaternary salts of N,N-dimethylaminoethyl acrylate, acrylic acid, various alkyl acrylates, methacrylic acid, various alkyl methacrylates, N-vinylpyrrolidone, acrylonitrile, styrene, polyethyleneglycol diacrylate, 1,6-hexanediol diacrylate, neopentyl glycol diacrylate, tripropylene glycol diacrylate, polypropylene glycol diacrylate, 2,2-bis[4-(acryloxydiethoxy)phenyl]propane, 2,2-bis[4-(acryloxypolyethoxy)phenyl]propane, 2,2-bis[4-(acryloxypolypropoxy)phenyl]propane, ethylene glycol dimethacrylate, diethylene glycol dimethacrylate, triethylene glycol dimethacrylate, polyethylene glycol dimethacrylate, 1,3-butylene glycol dimethacrylate, 1,6-hexanediol dimethacrylate, neopentyl glycol dimethacrylate, polypropylene glycol dimethacrylate, 2,2-bis[4-(methacryloxyethoxy)phenyl]propane, 2,2-bis[4-(methacryloxyethoxydiethoxy)phenyl]propane, 2,2-bis[4-(methacryloxyethoxypolyethoxy)phenyl]propane, trimethylolpropane trimethacrylate, tetramethylolmethane trimethacrylate, trimethylolpropane triacrylate, tetramethylolmethane triacrylate, tetramethylolmethane tetraacrylate, dipentaerythritol hexaacrylate, N,N'-methylene bisacrylamide, N,N'-methylene bismethacrylamide, diethylene glycol diallyl ether, divinylbenzene, ethylenebisacrylamide, N-[3-(dimethylamino)propyl]methacrylamide, diacetone acrylamide, N-t-butylacrylamide, N,N-diethylacrylamide, N-isopropylmethacrylamide, N-(butoxymethyl)acrylamide, N-(isobutoxymethyl)acrylamide, N-phenylacrylamide, 2-acrylamide-2-methylpropanesulfonic acid, 4-acrylamidobenzo-18-crown 6-ether, acryloylaminobenzocrown ether, methacryloylaminobenzocrown ether, and 4-vinylbenzocrown ether, and it is possible to use one member or two or more members in combination selected from these.

The content of the monomer having no OH group in the first polymer is preferably 0.1 mol% or more and 40 mol% or less, more preferably 0.2 mol% or more and 30 mol% or less, further more preferably 0.3 mol% or more and 20 mol% or less.

The first polymer may contain a crosslinking agent component as a constituent component.

According to this, the first polymer has a crosslinked structure, and thus has a three-dimensional network structure. As a result, the ability to retain the solvent of the stimulus-responsive gel can be made particularly excellent, and thus, a favorable gel state can be stably maintained over a long period of time. That is, the stimulus-responsive gel has excellent durability.

As the crosslinking agent component, a compound having two or more polymerizable functional groups can be used, and specific examples thereof include ethylene glycol, propylene glycol, trimethylol propane, glycerin, polyoxyethylene glycol, polyoxypropylene glycol, polyglycerin, N,N'-methylene bisacrylamide, N,N-methylene-bis-N-vinylacetamide, N,N-butylene-bis-N-vinylacetamide, tolylene diisocyanate, hexamethylene diisocyanate, allylated starch, allylated cellulose, diallyl phthalate, tetraallyloxyethane, pentaerythritol triallyl ether, trimethylol propane triallyl ether, diethylene glycol diallyl ether, and triallyl trimellitate, and it is possible to use one member or two or more members in combination selected from these.

The content of the crosslinking agent component in the first polymer is preferably 0.5 mol% or more and 7.0 mol% or less, more preferably 0.8 mol% or more and 6.0 mol% or less, further more preferably 1.1 mol% or more and 5.0 mol% or less.

According to this, the degree of crosslinking of the first polymer can be made to fall within a more favorable range, and while more remarkably exhibiting the effect as described above, the flexibility of the first polymer can be made more suitable.

The content of the monomer having an OH group in the first polymer is preferably 54 mol% or more and 99 mol% or less, more preferably 65 mol% or more and 98.5 mol% or less, further more preferably 76 mol% or more and 98 mol% or less.

According to this, while more remarkably exhibiting the effect of the first polymer because of having an OH group as described above, the effect of the components (the crosslinking agent, the monomer having no OH group, etc.) other than the monomer having an OH group can be sufficiently exhibited.

The hydroxyl value of the first polymer is preferably 15 mgKOH/g or more and 620 mgKOH/g or less, more preferably 34 mgKOH/g or more and 78 mgKOH/g or less.

According to this, while more remarkably exhibiting the effect of the first polymer because of having an OH group as described above, the durability of the stimulus-responsive gel can be made particularly excellent.

On the other hand, when the hydroxyl value of the first polymer is less than the above lower limit, the effect of the first polymer because of having an OH group as described above may not be sufficiently obtained depending on the percentage or the like of the phenylboronic acid structure possessed by the second polymer.

Further, when the hydroxyl value of the first polymer exceeds the above upper limit, the durability of the stimulus-responsive gel is decreased.

The first polymer does not have a phenylboronic acid structure.

The content X₁ of the first polymer in the stimulus-responsive gel is preferably 0.05 mass% or more and 98 mass% or less, more preferably 0.1 mass% or more and 70 mass% or less.

According to this, the sensitivity and quantitativeness with respect to lactic acid can be made particularly excellent, and also the ability to retain the solvent of the stimulus-responsive gel can be made particularly excellent, and thus, a favorable gel state can be stably maintained over a long period of time.

In addition, the content of the first polymer in the polymer material is preferably 1.0 mass% or more and 99 mass% or less, more preferably 1. 5 mass% or more and 98 mass% or less.

According to this, particularly excellent sensitivity and quantitativeness with respect to lactic acid are obtained, and also the ability to retain the solvent of the stimulus-responsive gel can be made particularly excellent, and thus, a favorable gel state can be stably maintained over a long period of time.

### Second Polymer

The second polymer has a phenylboronic acid structure.

By including such a second polymer along with the above-mentioned first polymer, the detection of the strength of a stimulus (the concentration or the like of a given component) in a wide range can be easily and stably performed. In particular, in the case where the sensor is used for performing the detection and quantitative determination of lactic acid, the sensitivity in a low concentration range (for example, in a range of 0.4 mass% or less) can be made excellent.

The phenylboronic acid structure may be a phenylboronic acid structure introduced after polymerizing a polymerizable compound (a monomer or the like) as a constituent component of the polymer, but is preferably a phenylboronic acid structure possessed by a polymerizable compound as a constituent component of the polymer.

According to this, the adjustment of the percentage or the like of the phenylboronic acid structure possessed by the second polymer can be easily and reliably performed.

Examples of the monomer having a phenylboronic acid structure which constitutes the second polymer include 3-acrylamidophenylboronic acid, vinylphenylboronic acid, acryloyloxyphenylboronic acid, acryloylaminobenzeneboronic acid, methacryloylaminobenzeneboronic acid, and 4-vinylbenzeneboronic acid, and it is possible to use one member or two or more members in combination selected from these, however, the second polymer preferably contains 3-acrylamidophenylboronic acid as a constituent component.

According to this, the transformation between the first state and the second state according to the change in the environment (particularly the change in the concentration of lactic acid) in which the stimulus-responsive gel is placed is more favorably performed, and the detection and quantitative determination of the strength of a stimulus (particularly, the concentration of lactic acid) can be more stably performed in a wider range. In addition, the ability to retain the solvent of the stimulus-responsive gel can be made particularly excellent, and thus, a favorable gel state can be stably maintained over a long period of time.

The second polymer may contain a monomer having no phenylboronic acid structure as a constituent component thereof. According to this, the percentage or the like of the phenylboronic acid structure possessed by the second polymer can be adjusted to a favorable level.

Examples of the monomer having no phenylboronic acid structure which constitutes the second polymer include acrylamide, N-methylacrylamide, N-isopropylacrylamide, N,N-dimethylacrylamide, N,N-dimethylaminopropyl acrylamide, various quaternary salts of N,N-dimethylaminopropyl acrylamide, acryloylmorpholine, various quaternary salts of N,N-dimethylaminoethyl acrylate, acrylic acid, various alkyl acrylates, methacrylic acid, various alkyl methacrylates, N-vinylpyrrolidone, acrylonitrile, styrene, polyethyleneglycol diacrylate, 1,6-hexanediol diacrylate, neopentyl glycol diacrylate, tripropylene glycol diacrylate, polypropylene glycol diacrylate, 2,2-bis[4-(acryloxydiethoxy)phenyl]propane, 2,2-bis[4-(acryloxypolyethoxy)phenyl]propane, 2,2-bis[4-(acryloxypolypropoxy)phenyl]propane, ethylene glycol dimethacrylate, diethylene glycol dimethacrylate, triethylene glycol dimethacrylate, polyethylene glycol dimethacrylate, 1,3-butylene glycol dimethacrylate, 1,6-hexanediol dimethacrylate, neopentyl glycol dimethacrylate, polypropylene glycol dimethacrylate, 2,2-bis[4-(methacryloxyethoxy)phenyl]propane, 2,2-bis[4-(methacryloxyethoxydiethoxy)phenyl]propane, 2,2-bis[4-(methacryloxyethoxypolyethoxy)phenyl]propane, trimethylolpropane trimethacrylate, tetramethylolmethane trimethacrylate, trimethylolpropane triacrylate, tetramethylolmethane triacrylate, tetramethylolmethane tetraacrylate, dipentaerythritol hexaacrylate, N,N'-methylene bisacrylamide, N,N'-methylene bismethacrylamide, diethylene glycol diallyl ether, divinylbenzene, ethylenebisacrylamide, N-[3-(dimethylamino)propyl]methacrylamide, diacetone acrylamide, N-t-butylacrylamide, N,N-diethylacrylamide, N-isopropylmethacrylamide, N-(butoxymethyl)acrylamide, N-(isobutoxymethyl)acrylamide, N-phenylacrylamide, N-hydroxyethylacrylamide, 2-hydroxyethylmethacrylate, glycerol monomethacrylate, 2-hydroxy-1-acryloxy-3-methacryloxypropane, 2-hydroxy-1,3-dimethacryloxypropane, 4-acrylamidobenzo-18-crown 6-ether, acryloylaminobenzocrown ether, methacryloylaminobenzocrown ether, and 4-vinylbenzocrown ether, and it is possible to use one member or two or more members in combination selected from these, however, the second polymer preferably contains N-hydroxyethylacrylamide as a constituent component.

According to this, the affinity between the first polymer and the second polymer can be made more favorable, and a problem of undesirable phase separation or the like in the stimulus-responsive gel can be more reliably prevented over a longer period of time, and the durability and reliability of the stimulus-responsive gel can be made particularly excellent. In addition, the ability to retain the solvent of the stimulus-responsive gel can be made particularly excellent.

The content of the monomer having no phenylboronic acid structure in the second polymer is preferably 1 mol% or more and 96 mol% or less, more preferably 5 mol% or more and 95 mol% or less, further more preferably 20 mol% or more and 93 mol% or less.

The second polymer may contain a crosslinking agent component as a constituent component.

According to this, the second polymer has a crosslinked structure, and thus has a three-dimensional network structure. As a result, the ability to retain the solvent of the stimulus-responsive gel can be made particularly excellent, and thus, a favorable gel state can be stably maintained over a long period of time. That is, the stimulus-responsive gel has excellent durability.

As the crosslinking agent component, a compound having two or more polymerizable functional groups can be used, and specific examples thereof include ethylene glycol, propylene glycol, trimethylol propane, glycerin, polyoxyethylene glycol, polyoxypropylene glycol, polyglycerin, N,N'-methylene bisacrylamide, N,N-methylene-bis-N-vinylacetamide, N,N-butylene-bis-N-vinylacetamide, tolylene diisocyanate, hexamethylene diisocyanate, allylated starch, allylated cellulose, diallyl phthalate, tetraallyloxyethane, pentaerythritol triallyl ether, trimethylol propane triallyl ether, diethylene glycol diallyl ether, and triallyl trimellitate, and it is possible to use one member or two or more members in combination selected from these.

The content of the crosslinking agent component in the second polymer is preferably 0.5 mol% or more and 10.0 mol% or less, more preferably 0. 8 mol% or more and 8.0 mol% or less, further more preferably 1.1 mol% or more and 6.0 mol% or less.

According to this, the degree of crosslinking of the second polymer can be made to fall within a more favorable range, and while more remarkably exhibiting the effect as described above, the flexibility of the second polymer can be made more suitable.

The content of the monomer having a phenylboronic acid structure in the second polymer is preferably 3.0 mol% or more and 98 mol% or less, more preferably 3.5 mol% or more and 70 mol% or less, further more preferably 3.8 mol% or more and 70 mol% or less.

According to this, the stimulus-responsive gel has particularly excellent flexibility, and therefore has particularly excellent sensitivity and quantitativeness with respect to a given stimulus, and the detection and quantitative determination of the strength of a stimulus (particularly, the concentration of lactic acid) can be more stably performed in a wider range.

On the other hand, when the content of the monomer having a phenylboronic acid structure in the second polymer is less than the above lower limit, it may be difficult to make the range (for example, the concentration of lactic acid) in which the detection and quantitative determination of the strength of a stimulus (for example, the concentration of lactic acid) can be favorably performed sufficiently wide depending on the percentage or the like of the OH group possessed by the first polymer.

In addition, when the content of the monomer having a phenylboronic acid structure in the second polymer exceeds the above upper limit, the stimulus-responsive gel is hard to deform, and therefore, the sensitivity and quantitativeness with respect to a given stimulus are deteriorated.

This is considered to be because the percentage of the phenylboronic acid structure is increased, so that the Π-Π electron interaction (Π-Π stacking) between a plurality of benzene rings strongly appears, and therefore a space into which the solvent or the like enters is reduced.

The content X₂ of the second polymer in the stimulus-responsive gel is preferably 0.01 mass% or more and 70 mass% or less, more preferably 0.05 mass% or more and 65 mass% or less.

According to this, the stimulus-responsive gel has particularly excellent flexibility, and therefore has particularly excellent sensitivity and quantitativeness with respect to a given stimulus, and the detection and quantitative determination of the strength of a stimulus (particularly, the concentration of lactic acid) can be more stably performed in a wider range.

On the other hand, when the content X₂ of the second polymer in the stimulus-responsive gel is less than the above lower limit, it may be difficult to make the range (for example, the concentration of lactic acid) in which the detection and quantitative determination of the strength of a stimulus (for example, the concentration of lactic acid) can be favorably performed sufficiently wide depending on the percentage or the like of the OH group possessed by the first polymer.

In addition, when the content X₂ of the second polymer in the stimulus-responsive gel exceeds the above upper limit, the stimulus-responsive gel is hard to deform, and therefore, the sensitivity and quantitativeness with respect to a given stimulus are deteriorated.

Further, the content of the second polymer in the polymer material is preferably 1.0 mass% or more and 70 mass% or less, more preferably 2. 0 mass% or more and 65 mass% or less.

According to this, the stimulus-responsive gel has particularly excellent flexibility, and therefore has particularly excellent sensitivity and quantitativeness with respect to a given stimulus, and the detection and quantitative determination of the strength of a stimulus (particularly, the concentration of lactic acid) can be more stably performed in a wider range.

On the other hand, when the content of the second polymer in the polymer material is less than the above lower limit, it may be difficult to make the range (for example, the concentration of lactic acid) in which the detection and quantitative determination of the strength of a stimulus (for example, the concentration of lactic acid) can be favorably performed sufficiently wide depending on the percentage or the like of the OH group possessed by the first polymer.

In addition, when the content of the second polymer in the polymer material exceeds the above upper limit, the stimulus-responsive gel is hard to deform, and therefore, the sensitivity and quantitativeness with respect to a given stimulus are deteriorated.

When the content of the first polymer in the stimulus-responsive gel is represented by X₁ (mass%) and the content of the second polymer in the stimulus-responsive gel is represented by X₂ (mass%), X₁ and X₂ preferably satisfy the following relationship: 0.2 ≤ X₂/X₁ ≤ 8, more preferably satisfy the following relationship: 1.3 ≤ X₂/X₁ ≤ 1.9.

According to this, the stimulus-responsive gel has particularly excellent flexibility, and therefore has particularly excellent sensitivity and quantitativeness with respect to a given stimulus, and the detection and quantitative determination of the strength of a stimulus (particularly, the concentration of lactic acid) can be more stably performed in a wider range.

On the other hand, when the value of X₂/X₁ is less than the above lower limit, it may be difficult to make the range (for example, the concentration of lactic acid) in which the detection and quantitative determination of the strength of a stimulus (for example, the concentration of lactic acid) can be favorably performed sufficiently wide.

In addition, when the value of X₂/X₁ exceeds the above upper limit, the stimulus-responsive gel is hard to deform, and therefore, the sensitivity and quantitativeness with respect to a given stimulus are deteriorated.

The content of the polymer material in the stimulus-responsive gel is preferably 0.7 mass% or more and 36.0 mass% or less, more preferably 2.4 mass% or more and 27.0 mass% or less.

### Solvent

By including the solvent in the stimulus-responsive gel, the above-mentioned polymer material can be favorably gelled.

As the solvent, any of various types of organic solvents and inorganic solvents can be used. Specific examples thereof include water; various types of alcohols such as methanol and ethanol; ketones such as acetone; ethers such as tetrahydrofuran and diethyl ether; amides such as dimethylformamide; chain aliphatic hydrocarbons such as n-pentane, n-hexane, n-heptane, and n-octane; alicyclic hydrocarbons such as cyclohexane and methylcyclohexane; and aromatic hydrocarbons such as benzene, toluene, and xylene, however, in particular, a solvent containing water is preferred.

The stimulus-responsive gel may contain a plurality of different types of components as the solvent.

The content of the solvent in the stimulus-responsive gel is preferably 30 mass% or more and 95 mass% or less, more preferably 50 mass% or more and 90 mass% or less.

### Another Component

The stimulus-responsive gel may contain a component other than the above-mentioned components (another component).

For example, the stimulus-responsive gel may contain particles having an average particle diameter of 10 nm or more and 1000 nm or less.

According to this, the structural color caused by Bragg reflection can be visually recognized. In particular, a distance between adjacent particles changes accompanying the expansion or contraction of the stimulus-responsive gel, and the structural color caused by Bragg reflection also changes, and therefore, not only can the stimulus be detected by measuring a resistance value as described above, but also the stimulus can be detected optically (visually). In addition, since the structural color caused by Bragg reflection or the change in the structural color is visually recognized, for example, by the color tone thereof, also the quantitative determination of a given stimulus (for example, a specific component) can be more easily and also more accurately performed.

### Application of Sensor

The sensor can easily detect a given stimulus (for example, a specific component), and therefore can be used as, for example, a sensor for determining whether or not a specific substance is contained in a test subject (a specimen) or measuring the concentration of a specific substance contained a test subject.

Further, the sensor can easily detect the amount of a specific component incorporated in the stimulus-responsive gel, and therefore can also be favorably used as a separation and extraction unit that separates and extracts a specific substance contained in a test subject. That is, at a stage where the amount of a specific component incorporated in the stimulus-responsive gel is saturated or almost saturated, the contact thereof with the test subject is stopped, and according to need, it can be replaced by another sensor. According to this, the specific component can be collected from the test subject without any waste.

More specific application of the sensor include, for example, sensors for biological substances (for example, various types of cells such as cancer cells and blood cells, proteins such as antibodies (including glycoproteins and the like), etc.), sensors for components (for example, lactic acid, uric acid, sugars, etc.) contained in body fluids or substances secreted outside the body (for example, blood, saliva, sweat, urine, etc.), separation and extraction units for biological substances (particularly, trace biological substances and the like such as hormones), separation and extraction units for metals (particularly, rare metals, noble metals, etc.), sensors for antigens (allergic substances) such as pollens, separation and extraction units for poisons, toxic substances, environmental pollutants, etc., sensors for viruses, bacteria, etc., sensors for components contained in soils, sensors for components contained in waste fluids (including drained water), sensors for components contained in foods, sensors for components contained in water (for example, salts and the like contained in brackish waters, rivers, paddies, etc.), cell culture monitors, and the like.

Further, the sensor is preferably a sensor to be used in close contact with the skin of a living body.

The skin of a living body generally has a complicated rugged shape, however, as described above, the sensor has excellent shape followability, and therefore can be favorably brought into close contact with the skin of a living body. Further, in the case where the sensor is used in close contact with the skin of a living body (for example, a case where a component contained in sweat when an exercise is performed is detected as a stimulus (specific component), or the like), it is assumed that a large external force such as vibration or impact is applied to the sensor. However, even in such a case that a relatively large external force is applied to the sensor, a specific component (given stimulus) can be accurately detected. Therefore, the effect is more remarkably exhibited in the case where the sensor is used in close contact with the skin of a living body.

Further, the sensor can be also favorably applied to reduction in size and weight. Accordingly, the sensor is suitable for use in the method as described above.

### Method of Using Sensor

Hereinafter, one example of a method of using the sensor will be specifically described.

First, the sensor 100 when the stimulus-responsive gel 11 is in a state where it does not receive a given stimulus (for example, in the case where the given stimulus is a specific component, a state where the stimulus-responsive gel 11 does not contain the specific component) is prepared.

Subsequently, the electrodes 30 and 40 are electrically connected to a unit that measures an electrical resistance, and the resistance value in a state where the stimulus-responsive gel 11 does not receive a given stimulus is measured (initialization). At this time, in the case where the given stimulus is a specific substance, for example, calibration may be performed using standard solutions containing the specific substance at given concentrations. By doing this, the stimulus detection accuracy can be further enhanced.

Thereafter, the detection of the stimulus is performed in a state where the specimen and the sensor 100 can come into contact with each other.

By performing the measurement in such a manner, the detection of the strength of a stimulus (the concentration or the like of a given component) can be easily and stably performed in a wide range.

The sensor 100 is preferably a sensor which constitutes a part of a wearable device or a sensor which is used by being connected to a wearable device.

According to this, for example, the burden on a user accompanying the detection of a stimulus can be reduced, and the sensor 100 can be favorably used, for example, even when an exercise is performed or the like. In addition, a user or the like can favorably confirm the detection result displayed on the display section. Further, such a configuration is preferred also from the viewpoint of fashion or the like.

Examples of the wearable device include a watch-type device.

In addition, for example, after the detection of a stimulus, the gel for a sensor 10 may be washed as needed. By doing this, the gel for a sensor 10 can be favorably reused, and the life of the gel for a sensor 10 and the sensor 100 can be prolonged. The washing of the gel for a sensor 10 can be performed using, for example, a liquid which does not contain a specific component (given stimulus).

When the sensor 100 is not used, the gel for a sensor 10 may be stored in a state where the gel for a sensor 10 is brought into contact with a liquid which does not contain a specific component (given stimulus). By doing this, the gel for a sensor 10 can be favorably stored, and the life of the gel for a sensor 10 and the sensor 100 can be prolonged.

Hereinabove, preferred embodiments have been described, however, the invention is not limited thereto.

For example, the sensor may have a configuration other than those described above.

For example, the sensor may include an adhesive layer for adhering the sensor to a given position.

In the above-mentioned embodiments, a case where the electrode is in contact with the gel for a sensor has been described, however, at least one intermediate layer may be provided between the electrode and the gel for a sensor. According to this, for example, the adhesiveness between the gel for a sensor and the electrode can be made more excellent.

Further, in the above-mentioned embodiments, a case where the electrodes are provided on the side of the face of the electrically conductive gel opposite to the face on the side facing the stimulus-responsive gel has been representatively described, however, the place where the electrodes are provided is not limited thereto. For example, the electrodes may be provided in a side face portion of the electrically conductive gel in the form of a sheet. According to this, the thickness of the sensor can be reduced. In such a case, the electrodes may be provided only in a portion in the thickness direction of the gel for a sensor or may be provided throughout the thickness direction of the gel for a sensor.

Further, in the above-mentioned embodiments, a case where the stimulus-responsive gel and the electrically conductive gel are in contact with each other has been representatively described, however, for example, at least one intermediate layer may be provided between the stimulus-responsive gel and the electrically conductive gel.

Further, the sensor may have a partition that divides the gel for a sensor into a plurality of cells.

Further, in the above-mentioned embodiments, a case where the electrically conductive gel is in the form of a sheet has been representatively described, however, the electrically conductive gel may be in any form other than a sheet, and similarly, the shape of the stimulus-responsive gel may also be not particularly limited.

In addition, in the sensor, some constituent members may be configured to be replaceable or may be configured to be detachable and reattachable. For example, the absorbing member may be configured to be replaceable or detachable and reattachable. According to this, even if a large amount of a solid component (for example, a specific component or the like) is adhered to the absorbing member, by replacing the absorbing member, or detaching and washing the absorbing member, or the like, the sensor can be used. In this manner, the life of the sensor can be prolonged.

## Claims

1. A gel for a sensor, comprising:
a stimulus-responsive gel which expands or contracts in response to a given stimulus; and
an electrically conductive gel containing electrically conductive particles constituted by a material containing an electrically conductive substance.

2. The gel for a sensor according to claim 1, wherein the average particle diameter of the electrically conductive particles is 10 nm or more and 1000 µm or less.

3. The gel for a sensor according to claim 1 or 2, wherein the electrical resistivity of the electrically conductive substance is 1.0×10⁻³ Ω·m or less.

4. The gel for a sensor according to any one of the preceding claims, wherein the electrically conductive substance is constituted by a material containing one or more members selected from the group consisting of a metal material, an electrically conductive metal oxide, a carbon material, and an electrically conductive polymer material.

5. The gel for a sensor according to claim 1, wherein the electrically conductive gel is provided with a recessed portion in a region coming into contact with an electrode.

6. The gel for a sensor according to claim 5, wherein in the recessed portion, the electrically conductive particles and the electrode are in contact with each other.

7. A sensor, comprising:
the gel for a sensor according to any one of the preceding claims; and
an electrode connected to the electrically conductive gel.

8. A sensor, comprising:
the stimulus-responsive gel reversibly expands or contracts in response to a given stimulus.

9. The sensor according to claim 7 or 8, further comprising a housing member having a housing portion in which the stimulus-responsive gel and the electrically conductive gel are housed.

10. The sensor according to claim 9, wherein the housing member is constituted by a material which is harder than the stimulus-responsive gel and the electrically conductive gel.

11. The sensor according to claim 10 or 11, wherein the housing member is provided with an inflow port for allowing a specimen to flow in the housing portion.

12. The sensor according to any one of claims 7 to 11, wherein when the stimulus-responsive gel expands, a pressing force acts on the electrically conductive gel in the stacking direction of the stimulus-responsive gel and the electrically conductive gel.

13. The sensor according to any one of claims 7 to 12, wherein the stimulus-responsive gel is configured so that when the stimulus-responsive gel is deformed from a contracted state to an expanded state, the percentage of deformation thereof toward the direction in which the electrically conductive gel is provided is larger than the percentage of deformation thereof toward the other directions.

14. The sensor according to any one of claims 7 to 13, wherein the sensor is used in a state where the stimulus-responsive gel is disposed on the side where a specimen is supplied closer than the electrically conductive gel.

15. The sensor according to any one of claims 7 to 14, wherein the electrode is disposed on the side of the face of the electrically conductive gel opposite to the face on the side facing the stimulus-responsive gel.

16. The sensor according to any one of claims 7 to 15, wherein the width of the face of the stimulus-responsive gel facing the electrically conductive gel is smaller than the length in the normal direction of the face.

17. The sensor according to any one of claims 7 to 16, wherein the stimulus-responsive gel has a portion with a tapered cross-sectional area, in which the cross-sectional area of the portion is gradually reduced from the face on the side where the specimen is supplied to the face on the opposite side.

18. The sensor according to any one of claims 7 to 17, wherein when a region where the stimulus-responsive gel and the electrically conductive gel are in contact with each other is seen in a plan view from the normal direction of the contact face of the electrically conductive gel with the stimulus-responsive gel, the region is overlapped with a range including at least a portion of a region between the electrodes.
